# EUROPEAN PATENT APPLICATION

(11) **EP 1 489 416 A1**
(43) Date of publication of application: **22.12.2004**
(21) Application number: 03712791.7
(22) Date of filing: 20.03.2003
(51) Int. Cl.: G01N 33/53, G01N 33/569, G01N 33/68

(54) **METHOD OF JUDGING VIRAL INFECTION**

(30) Priority: 22.03.2002 JP 2002080324
(71) Applicant: KYOWA MEDEX CO., LTD., Tokyo 104-0042 (JP)
(72) Inventor: MIYAWAKI, Toshio, Toyama-shi, Toyama 930-0876 (JP)
(74) Representative: VOSSIUS & PARTNER
(86) International application number: PCT/JP2003/003408
(87) International publication number: WO 2003/081240

(57) **Abstract**

A method for diagnosing a viral infection without complicating bacterial infection, which comprises determining a C-reactive protein and an MxA protein in a biological sample; a kit for judging viral infection without complicating bacterial infection, which comprises a reagent for determining a C-reactive protein and a reagent for determining an MxA protein in a biological sample.

## Description

### Technical Field

The present invention relates to a method for diagnosing viral infections and a kit for the judgment.

### Background Art

Since the discovery of penicillin in 1928, it has become possible to treat considerable bacterial infections by antibiotics which have been developed and improved one after another. However, this fact does not mean that all infections including bacterial infections can be treated. Many patients suffering from infections still need to be treated and hence it has been desired to develop a diagnostic method for selecting a treatment method. However, there exists no clear and rapid method for distinction between bacterial infections and viral infections in clinical practice at the present stage.

Until now, for the distinction of bacterial infections and other infections, symptoms in patients, the number of peripheral leukocytes and C-reactive protein values are employed. The C-reactive protein is one of acute phase response substances and is known as a marker for diagnosis and progress observation of various infections. The C-reactive protein value in blood of a healthy adult is 0.06 mg/dL. The case where the C-reactive protein value in blood is from 1 to 10 mg/dL is diagnosed to be a disorder of medium-grade increase and the case where the value is 10 mg/dL or higher is diagnosed to be a disorder of high-grade increase. When the diagnosis is a disorder of medium-grade increase, chronic rheumatism, angiitis, rheumatic fever, malignant tumor, cardiac infarction, and external injury are suspected in addition to bacterial infections. Moreover, when the diagnosis is a disorder of high-grade increase, serious bacterial infections and active rheumatism are suspected [*Clinical Test Method Summary (Rinsho Kensa Ho Teiyo),* published by Kanehara Shuppan, 500 (1998)].

As mentioned above, it is difficult to diagnose bacterial infections perfectly with the C-reactive protein value in blood which is employed for diagnosis of bacterial infections.

Recently, MxA proteins have attracted attention as specific markers for viral infections. MxA proteins are induced in lymphocytes by type I interferons (IFN-α and IFN-β) which are increased at viral infection, but are not at all affected by the other inflammatory cytokines such as IL-1, IL-6 and TNF-α of which productions are increased in bacterial infections and chronic inflammatory diseases [*Eur J Immunol,* 20, 2015 (1990)]. Therefore, the expression of an MxA protein in lymphocytes is considered to be utilized for detection of all viral infections and a possibility of its clinical application has been reported [*Pediatr Res,* 41, 647 (1997)].

In medical sites, there is a tendency that antibiotics are used for patients when they are suspected to suffer from bacterial infections [*JAMA,* 273, 213 (1995)]. Such use of antibiotics has resulted in a serious problem of occurrence of multidrug-resistant bacteria such as methicillin-resistant Staphylococcus aureus (MRSA) which are resistant to various antibiotics.

Apporopriate use of antibiotics is expected to be able to contribute not only to prevention of the appearance of multidrug-resistant bacteria but also to suppression of increase of medical costs which is currently recognized as a problem [*Pedicatrics,* 108, 1 (2001)]. Therefore, it is important to clearly distinguish between viral infections and bacterial infections, but the method to distinguish them has not been known until now.

### Disclosure of the Invention

An object of the present invention is to provide a method for diagnosing a viral infection without complicating bacterial infection and a reagent used for the diagnosis.

The present invention relates to a method for diagnosing a viral infection without complicating bacterial infection, which comprises determining a C-reactive protein and an MxA protein in a biological sample.

Moreover, the present invention relates to a kit for diagnosing a viral infection without complicating bacterial infection, which comprises a reagent for determining a C-reactive protein and a reagent for determining an MxA protein in a biological sample.

In order to diagnose a viral infection without complicating bacterial infection, the C-reactive protein and the MxA protein are measured.

The method for determining a C-reactive protein is not particularly limited, so long as it is a method for determining a C-reactive protein. Examples include an immunological determining method using an antibody against a C-reactive protein. As the immunological determining method, any method is included, so long as it uses an antigen-antibody reaction, such as an immunoassay, an immunoblotting method, an agglutination reaction, a complement-fixation reaction, a hemolytic reaction, a precipitation reaction, a gold colloid method, a chromatography method or an immunostaining method, and an immunoassay is preferred.

The immunoassay is a method for detecting or determining an antibody or antigen by using variously labeled antigens or antibodies. The method includes a radioimmunoassay (RIA), an enzyme immunoassay (EIA or ELISA), a fluorescent immunoassay (FIA), a luminescent immunoassay, a physicochemical assay (TIA, LAPIA, PCIA), a flow cytometry and the like, depending on the method of labeling the antigens or antibodies. As the method for determining a C-reactive protein, a physicochemical assay is preferred.

Specifically, the assay is carried out by binding a C-reactive protein as an antigen to an antibody or antiserum which specifically binds to the C-reactive protein to thereby form an agglutinate and by detecting the agglutinate. The other physicochemical assays include methods for determination using a capillary method, a one-dimensional immunodiffusion method, an immunonephelometry, a latex immunonephelometry or the like [*Clinical Test Method Summary (Rinsho Kensa Ho Teiyo),* published by Kanehara Shuppan, 499 (1998)].

For example, in the latex immunonephelometry, when an antigen-antibody reaction is induced on a polystyrene latex having a particle size of about 0.1 to 1 µm immobilized with an antibody or antigen by using a corresponding antigen or antibody, scattered light increases while transmitted light decreases in the reaction mixture. The C-reactive protein can be determined by measuring the change as absorbance or integrating sphere turbidity. Specific reagents for determining a C-reactive protein include Determiner T CRP and Extel CRP manufactured by Kyowa Medex Co., Ltd., N-Assay TIA-CRP-H Kit manufactured by Nitto Boseki Co., Ltd., and the like.

The method for determining an MxA protein is not particularly limited, so long as it is a method for determining an MxA protein. Examples include an immunological determining method using an antibody against an MxA protein. As the immunological determining method, any method can be used, so long as it uses an antigen-antibody reaction, such as an immunoassay, an immunoblotting method, an agglutination reaction, a complement-fixation reaction, a hemolytic reaction, a precipitation reaction, a gold colloid method, a chromatography method or an immunostaining method. As the method for determining an MxA protein, an immunoassay is preferred.

The immunoassay is a method for detecting or determining an antibody or antigen by using variously labeled antigens or antibodies. The method includes a radioimmunoassay (RIA), an enzyme immunoassay, (EIA or ELISA), a fluorescent immunoassay (FIA), a luminescent immunoassay, a physicochemical assay (TIA, LAPIA, PCIA), a flow cytometry and the like, depending on the method of labeling the antigen or antibody, and a flow cytometry is preferred.

As a fluorescent label used in a flow cytometry, any known fluorescent labels (Akira Kawaoi, *Fluorescent Antibody Method (Keiko Koutai Ho),* published by Soft Science Inc.) can be employed. For example, FITC, RITC and the like can be used.

Specifically, an antibody or antiserum specifically binding to an MxA protein is allowed to react with peripheral lymphocytes subjected to paraffin fixing, the antibody specifically bound to an MxA protein expressed in the lymphocytes is then allowed to react with a fluorescence-labeled appropriate secondary antibody, and difference of fluorescent intensity is detected by a flow cytometry [WO96/05230 *Allergy,* 56, 895 (2001)].

The kit for diagnosing a viral infection without complicating bacterial infection according to the present invention comprises a reagent for determining a C-reactive protein and a reagent for determining an MxA protein. The kit of the present invention may be a combination of individual independent kits of the reagent for determining a C-reactive protein and the reagent for determining an MxA protein as well as a kit containing the reagent for determining a C-reactive protein and the reagent for determining an MxA protein in one. Moreover, in the kit comprising the reagent for determining a C-reactive protein and the reagent for determining an MxA protein in one, common reagents of the respective reagents can be commonly used. The common reagents include a diluting solution for a biological sample, an antibody-immobilized solid phase, a reaction buffer, a washing liquid, a labeled secondary antibody or antibody fragment thereof, a reagent for detecting a label, and the like. The kit of the present invention can be combined with an instrument suitable for the measurement to form a further kit.

Even when the constitution or form is different, any kit is included in the kits of the present invention, so long as it contains substances essentially the same as individual constitutional elements of the reagents for determining a C-reactive protein or the reagents for determining an MxA protein or essentially the same as a part thereof.

The reagent for determining a C-reactive protein includes an antibody reactive to a C-reactive protein, and also include, if necessary, a diluting solution for a biological sample, an antibody-immobilized solid phase, a reaction buffer, a washing solution, a labeled secondary antibody or antibody fragment thereof, a reagent for detecting a label, a standard substance of a C-reactive protein, and the like.

The antibody reactive to a C-reactive protein is not particularly limited, so long as it is an antibody reactive to a C-reactive protein, and examples include commercially available anti-human C-reactive protein polyclonal antibodies of goat, rabbit, rat and the like, and a murine anti-human C-reactive protein monoclonal antibody (manufactured by SIGMA).

The diluting solution for a biological sample includes aqueous solutions containing proteins such as BSA or casein in addition to a surfactant, a buffer and the like.

The antibody-immobilized solid phase includes a solid phase wherein an antibody or antibody fragment is immobilized on a material obtainable by shaping various polymer materials so as to conform to the use. The shape includes a tube, beads, a plate, fine particles such as latex, a stick and the like. The material includes polymer materials such as polystyrene, polycarbonate, polyvinyltoluene, polypropylene, polyethylene, polyvinyl chloride, nylons, polymethacrylates, gelatin, agarose, cellulose and polyethylene terephthalate, glass, ceramics, metals and the like. As methods for immobilizing an antibody, known methods are employed, e.g., physical methods, chemical methods, and combination thereof. Examples include a solid phase obtainable by hydrophobically immobilizing an antibody or the like on a 96-well microtiter plate for immunoassay made of polystyrene.

The reaction buffer may be any buffer, so long as the antibody on the antibody-immobilized solid phase is capable of binding to the antigen in a biological sample, and examples include phosphate buffers, Good's buffer and the like. Moreover, if necessary, a surfactant, a protein such as BSA or casein, an antiseptic, a stabilizer, a reaction accelerator and the like can be added.

The washing solution includes a phosphate buffer saline (hereinafter referred to as "PBS") containing 0.05% Tween 20 and the like. Moreover, if necessary, a surfactant, a protein such as BSA or casein, an antiseptic, a stabilizer or the like can be added.

The labeled secondary antibody or antibody fragment thereof includes an antibody or antibody fragment obtained by binding a labeling enzyme such as horseradish peroxidase, bovine small intestine alkaline phosphatase or β-galactosidase to the antibody or antibody fragment. To the secondary antibody or antibody fragment thereof, if necessary, a buffer, a protein such as BSA or casein, an antiseptic or the like can be added.

As the reagent for detecting the label, according to the above labeling enzyme, for example, a substrate for absorption photometry, such as tetramethylbenzidine or o-phenylenediamine, a fluorescent substrate such as hydroxyphenyl hydroxyphenylpropionate or hydroxyphenylacetatic acid, or a luminescent substrate such as luminol can be used for horseradish peroxidase, and a substrate for absorption photometry such as 4-nitrophenyl phosphate, a fluorescent substrate such as 4-methylumbelliferyl phosphate, or the like can be used for alkaline phosphatase.

The standard substance includes C-reactive proteins obtained by recombinant DNA techniques or obtained from a biological sample, cells in which a C-reactive protein is expressed, and the like.

The reagent for determining an MxA protein includes an antibody reactive to an MxA protein, and also includes, if necessary, a diluting solution for a biological sample, an antibody-immobilized solid phase, a reaction buffer, a washing solution, a labeled secondary antibody or antibody fragment thereof, a reagent for detecting a label, a standard substance of an MxA protein, and the like.

The antibody reactive to an MxA protein is not particularly limited, so long as it is an antibody reactive to an MxA protein, and examples include an anti-MxA protein antibody KM1135 produced by a hybridoma FERM BP-4731 (WO96/05230).

The diluting liquid for a biological sample includes aqueous solutions containing proteins such as BSA or casein, in addition to a surfactant, a buffer and the like.

The antibody-immobilized solid phase includes a solid phase wherein an antibody or antibody fragment is immobilized to a material obtainable by shaping various polymer materials so as to conform to the use. The shape includes a tube, beads, a plate, fine particles such as latex, a stick, and the like. The material includes polymer materials such as polystyrene, polycarbonate, polyvinyltoluene, polypropylene, polyethylene, polyvinyl chloride, nylons, polymethacrylates, gelatin, agarose, cellulose and polyethylene terephthalate, glass, ceramics, metals, and the like. As methods for immobilizing an antibody, known methods are employed, e.g., physical methods, chemical methods, and combination thereof. Examples include a solid phase obtainable by hydrophobically immobilizing an antibody or the like on a 96-well microtiter plate for immunoassay made of polystyrene.

The reaction buffer to be incorporated in the reagent for determining an MxA protein may be any buffer, so long as the antibody of the antibody-immobilized solid phase is capable of binding to the antigen in a biological sample, and examples include a phosphate buffer, a Good's buffer and the like. Moreover, if necessary, a surfactant, a proteins such as BSA or casein, an antiseptic, a stabilizer, a reaction accelerator or the like can be added.

The washing solution may be any washing solution, so long as it is capable of washing those which do not participate in the antigen-antibody reaction for determining an MxA protein, and examples include fetal bovine serum and PBS containing 0.1% azide and the like. Moreover, if necessary, a buffer, a surfactant, a protein such as BAS or casein, an antiseptic, a stabilizer or the like can be added.

The labeled secondary antibody or antibody fragment thereof includes an antibody or antibody fragment obtainable by binding a labeling enzyme such as horseradish peroxidase, bovine small intestine alkaline phosphatase or β-galactosidase to the antibody or antibody fragment. To the secondary antibody or antibody fragment thereof, if necessary, a buffer, a protein such as BSA or casein, an antiseptic or the like can be added.

As the reagent for detecting the label, according to the above labeling enzyme, for example, a substrate for absorption photometry, such as tetramethylbenzidine or o-phenylenediamine, a fluorescent substrate such as hydroxyphenyl hydroxyphenylpropionate or hydroxyphenylacetic acid, or a luminescent substrate such as luminol can be used for horseradish peroxidase, and a substrate for absorption photometry such as 4-nitrophenyl phosphate, a fluorescent substrate such as 4-methylumbelliferyl phosphate, and the like can be used for alkaline phosphatase.

The standard substance includes MxA proteins obtained by recombinant DNA techniques or obtained from a biological sample, cells in which an MxA protein is expressed, and the like.

As the biological sample used in the present invention, for example, blood can be used.

In the present invention, the C-reactive protein and the MxA protein are determined and based on each observed value, the presence or absence of the expression of the C-reactive protein and the MxA protein is judged, so that a viral infection without complicating bacterial infection is diagnosed.

In this case, the case where the C-reactive protein is negative and the MxA protein is positive is diagnosed to be a viral infection without complicating bacterial infection. On the other hand, the case where the C-reactive protein is positive and the MxA protein is negative is diagnosed to be a bacterial infection without complicating viral infection. The case where the C-reactive protein is positive and the MxA protein is positive is diagnosed to be a viral infection with a bacterial infection. The case where the C-reactive protein is negative and the MxA protein is negative is diagnosed to be unknown.

In the present invention, the criteria for diagnosis as positive or negative on each observed value of the C-reactive protein and the MxA protein can be suitably decided depending on a determining method. For example, when the C-reactive protein in blood is measured by an immunoassay, the diagnosis is carried out by considering a value of less than 1.0 mg/dl to be negative and a value of 1.0 mg/dl or more to be positive. Moreover, when the MxA protein is measured by an immunoassay, an average value and standard deviation on healthy persons in whom no viral infection is observed are obtained, and the diagnosis is carried out by considering a value of the average value + 3SD or more to be positive and a value of less than the above value to be negative.

Specific examples are shown below.

### Best Mode for Carrying Out the Invention

### Example 1

The following measurements were carried out by using peripheral blood samples of 74 persons who were suspected to suffer from any of infections and from whom blood samples were collected for various assay.

The collected peripheral blood samples were immediately injected into serum-submitting spits tubes for determining a C-reactive protein value in blood and for the other general assay, and into a spits tube containing heparin Na for determining an MxA protein, and were treated within the day.

The C-reactive protein value in blood was measured by using N-ASSAY TIA-CRP-H kit manufactured by Nitto Boseki Co., Ltd. according to the attached protocol. The observed C-reactive protein value in blood was judged by using a value of 1.0 mg/dl as a standard value and by considering patients having a value of less than 1.0 mg/dl to be negative and patients having a value of 1.0 mg/dl or more to be positive,.

The expression of the MxA protein in lymphocytes was analyzed as follows.

After 200 µl of peripheral blood collected with heparin was fixed with 4% paraformaldehyde, erythrocytes were destroyed and lymphocytic membrane was made permeable with a lysis buffer containing 0.1% Triton X-100. Then, staining was carried out with the anti-MxA protein antibody KM1135 produced by a hybridoma cell FERM BP-4731 or a primary antibody of an anti-murine IgG1 antibody as a control antibody. After washing, color was developed by using a secondary antibody, and analysis was carried out with a flow cytometer, and the difference of the fluorescence intensity and that of the control antibody is considered to be an MxA protein value. Cord blood in normal childbirth was used for obtaining a standard value, and for convenience' sake, a fluorescence intensity of its average value + 3SD or more was used as a standard value. It was diagnosed that patients showing the average value + 3SD or less were negative for expression of an MxA protein in lymphocytes and patients showing the average value + 3SD or more was positive for expression of an MxA protein in lymphocytes.

With regard to a fever, patients having a maximum body temperature on the blood-collected day of 37.5°C or higher was judged to have a fever and patients having a maximum body temperature of 37.5°C or lower was judged to have no fever.

Table 1 shows results obtained by classifying these 74 patients under judgment based on a fever, judgment based on the C-reactive protein value in blood, and judgment based on the expression of the MxA protein in lymphocytes.

**Table 1**

| | Patients having a fever | Patients having no fever |
|---|---|---|
| Total number of patients | 48 | 26 |
| C-reactive protein value in blood (positive) | 31 (64.6%) | 6 (23.1%) |
| Expression of MxA protein in lymphocytes (positive) | 33 (68.8%) | 16 (61.5%) |

The ratios of the patients who were positive in the diagnosis based on the C-reactive protein value in blood were 64.6% in the case that they had a fever and 23.1% in the case where they had no fever. On the other hand, the ratios of the patients who were positive based on the expression of the MxA protein in lymphocytes were 68.8% in the case where they had a fever and 61.5% in the case where they had no fever. Therefore, the positive diagnosis based on the C-reactive protein value in blood and the positive diagnosis based on the expression of the MxA protein in lymphocytes were found to be attributable to independent judgment criteria and hence a possibility that much information was obtained by combining both of them was shown.

Actually, when 74 patients were analyzed by combining the diagnosis based on the C-reactive protein value in blood and the diagnosis based on the expression of the MxA protein in lymphocytes, there were some cases where causes of diseases could be identified in patients whose diseases had been diagnosed as simple bacterial infections or whose causes of the diseases had been unknown.

There were 31 patients who had a fever and were diagnosed to be positive based on the C-reactive protein value in blood, and their diseases had hitherto been diagnosed as bacterial infections. When the diagnosis based on the expression of the MxA protein in lymphocytes was combined for the 31 patients, patients who were positive in the expression of the MxA protein in lymphocytes were found to be 19 patients. Among the 19 patients, 5 cases of asthma attack and 5 cases of possible viral adenoiditis were included and thus existence of patients who were also considered to suffer from viral infections was revealed. This fact shows that the diagnosis method of the present invention is superior to the conventional diagnosis method wherein the case that a fever is observed and the based on the C-reactive protein value in blood is positive is diagnosed to be a bacterial infection.

Additionally, even when a fever was observed, the causes of 17 patients who were diagnosed to be negative based on the C-reactive protein value in blood had been hitherto diagnosed to unknown. When the diagnosis based on the expression of the MxA protein in lymphocytes was combined for the 17 patients, the patients who were diagnosed to be positive based on the expression of the MxA protein in lymphocytes were found to be 14 patients. Actually, among the 14 patients, 4 cases of asthma attack, 2 cases of viral gastroenteritis (suspected), and 2 cases of viral encephalitis (suspected) were included.

Patients who had no fever and were negative in the diagnosis based on the C-reactive protein value in blood and hence who were diagnosed not to suffer from bacterial infection were found to be 20 patients. When the diagnosis based on the expression of the MxA protein in lymphocytes was combined for the 20 patients, patients who were positive in the diagnosis based on the expression of the MxA protein in lymphocytes was found to be 14 patients.

Actually, among the 14 patients, viral infection was confirmed in 5 patients and it was confirmed that 4 patients suffered from RS virus infection and 1 person suffered from congenital cytomegalovirus infection.

Therefore, even in the cases that the judgment based on the C-reactive protein value in blood was negative and thus the causes of diseases were unknown in the past, it has been found that viral infections can easily be diagnosed and therapeutic strategy can be rapidly decided by combining the diagnosis based on the expression of the MxA protein in lymphocytes.

### Industrial Applicability

According to the present invention, a method for diagnosing a viral infection without complicating bacterial infection and a kit used for the method are provided.

A viral infection without complicating bacterial infection can be diagnosed by analyzing a C-reactive protein and an MxA protein simultaneously by using the method for diagnosing a viral infection without complicating bacterial infection and the kit used for the method according to the present invention.

## Claims

1. A method for diagnosing a viral infection without complicating bacterial infection, which comprises determining a C-reactive protein and an MxA protein in a biological sample.

2. A kit for diagnosing a viral infection without complicating bacterial infection, which comprises a reagent for determining a C-reactive protein and a reagent for determining an MxA protein in a biological sample.
